# EUROPEAN PATENT APPLICATION

(11) **EP 3 847 962 A1**
(43) Date of publication of application: **14.07.2021**
(21) Application number: 18932860.2
(22) Date of filing: 05.09.2018
(51) Int. Cl.: A61B 5/145, A61B 5/00, H04M 1/725

(54) **BLOOD GLUCOSE MEASUREMENT DEVICE AND BLOOD GLUCOSE MEASUREMENT SYSTEM USING SAME**

(71) Applicant: Kwangwoon University Industry-Academic Collaboration Foundation, Seoul 01897 (KR)
(72) Inventor: SHIM, Joon Sub, Yongin-si Gyeonggi-do 16902 (KR)
(74) Representative: De Vries & Metman
(86) International application number: PCT/KR2018/010359
(87) International publication number: WO 2020/050433

(57) **Abstract**

The present invention relates to: a blood glucose measurement device, which is a non-invasive blood glucose measurement device that measures blood glucose using sweat rather than blood, and is equipped with a sweat inducing means that does not require a drug during a sweat inducing process; and a blood glucose measurement system using same. The present invention provides a blood glucose measurement device and a blood glucose measurement system using same, wherein the blood glucose measurement device comprises: a sweat inducing unit having two electrodes which are disposed to be spaced apart from each other and to which opposite polarities are applied; a housing for fixing the sweat inducing unit such that the two electrodes are exposed on one surface thereof; and a blood glucose sensor which is inserted into the housing and comes into contact with bodily fluid produced by the sweat inducing unit. Thus, the blood glucose measurement device does not cause problems, such as pain, infection, and scarring, that can be caused by needles, and does not use drugs that can affect the normal sweat secretion activity structure of the skin, and thus does not have any aftereffects for the skin. Even if the amount of sweat secreted is small, the sweat is efficiently collected so that all of the collected sweat can be used for blood glucose measurement, making highly accurate blood glucose measurements possible. The present invention therefore has the effect in which frequent blood glucose monitoring, which is important for diabetic patients, can be performed without any aftereffects.

## Description

### Technical Field

The present invention relates to a blood glucose measurement device and a blood glucose measurement system using the same and, more particularly, to a blood glucose measurement device and a blood glucose measurement system using the same, the blood glucose measurement device being non-invasive, measuring blood glucose with sweat rather than blood, and being provided with a sweat-inducing means that requires no drug in a sweat induction process.

### Background Art

Diabetes is a disease old enough to be confirmed in ancient Egyptian records, but it may be said that the history of a blood glucose measurement device enabling patients to measure blood glucose by themselves began in 1965 when the first blood glucose test strip, Dextrosticx, was marketed.

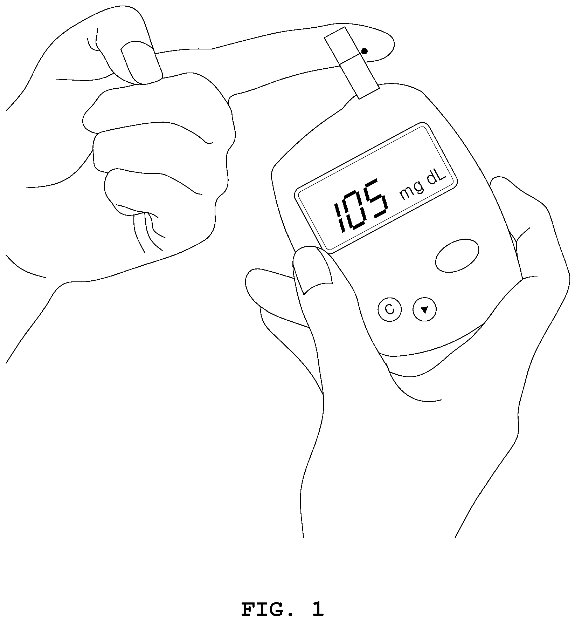

As shown in a view of FIG. 1, a blood glucose measurement device generally is composed by a structure in which blood glucose is measured with blood collected by puncturing the skin with a needle. However, in order to obtain a more accurate blood glucose measurement value, frequent blood glucose measurement should be performed. In this case, at each time, the blood collection causes a puncture on the skin due to the needle, so the pain caused by the needle increases as much as a patient is trying to measure blood glucose.

Due to this problem, a blood glucose measurement device capable of collecting blood with a micro-needle having a minimized needle diameter has been developed, and there is also a trend of developing a non-invasive blood glucose measurement device.

Compared to previous blood glucose measurement devices, the blood glucose measurement device using the micro-needle may lessen the pain, but since a needle is also used as before, there may occur a problem in that scars may remain due to frequent tests, and infection may be caused by the use of the needle.

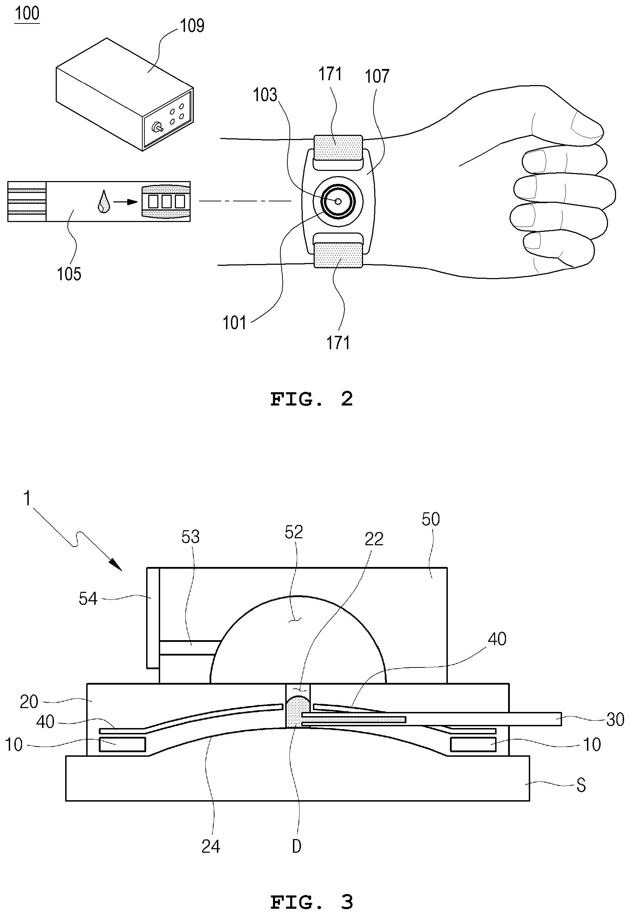

As presented in Korean Patent Application Publication No. 10-2016-0089718 (published date: July 28, 2016) "SENSOR MODULE AND WEARABLE ANALYZING DEVICE FOR BODY COMPOSITON WITH THE SAME", which is the related art shown in FIG. 2, a non-invasive blood glucose measurement device is provided with a sweat induction part 101 and a sweat collection part 103, wherein the sweat gland control nerves are stimulated by infiltrating the skin with drugs such as pilocarpine or acetylcholine that may induce sweat secretion from the skin, and the secreted sweat is collected so as to check a level of blood glucose contained in the sweat.

Meanwhile, the chemical stimulation for inducing sweat secretion is so strong that drug resistance may be caused in the user's skin. Accordingly, as the non-invasive blood glucose measurement device using drugs to more accurately measure blood glucose levels is frequently used, abnormalities may occur in the normal sweat-inducing mechanism in an increasingly larger area of the skin.

Nevertheless, as for diabetic patients, more accurate blood glucose management is possible only when more frequent blood glucose measurements are performed at times, such as before and after meals, and after a certain period of time from a meal time. In other words, due to a meal, as a satiety state and a fasting state occur alternately over time, the level of blood glucose also changes significantly, so it is difficult to obtain an accurate blood glucose measurement value with several intermittent blood glucose measurements. Accordingly, as needed, frequent blood glucose measurements should be performed. However, in currently developed blood glucose measurement devices, an invasive blood glucose measurement device has a problem in that pain, infection, and scars are caused, and a non-invasive blood glucose measurement device has a concern in that drug resistance to the skin and an abnormal sweat secretion mechanism caused by drug administration may occur, and thus it is difficult for such frequent blood glucose measurements to be performed.

Therefore, there is an urgent need to develop a blood glucose measurement device that uses a non-invasive method causing no problem of pain and infection, and does not use drugs that may cause skin abnormalities.

### Documents of Related Art

Korean Patent Application Publication No. 10-2016-0089718 (published date: July 28, 2016)

### Disclosure

### Technical Problem

Accordingly, the present invention is to solve the problems of the related art, and an objective of the present invention is to provide a blood glucose measurement device that uses a non-invasive method causing no problem of pain and infection, and does not use drugs that may cause abnormalities such as sequelae or drug resistance to the skin.

### Technical Solution

According to the present invention for achieving this objective, a blood glucose measurement device includes: a sweat-inducing part having two electrodes arranged to be spaced apart from each other and to which opposite polarities are respectively applied; a housing for fixing the sweat-inducing part allowing the two electrodes to be exposed on one surface of the housing; and a blood glucose sensor inserted into the housing and in contact with body fluids induced by the sweat-inducing part.

Here, preferably a heater may be installed inside the housing close to the one surface of the housing, and in this case, the heater may preferably have a micro hot wire, or a Peltier element, or a PTC element.

In addition, preferably, the blood glucose sensor may be detachably inserted into the housing from an outer side of the housing.

Meanwhile, preferably, a fine channel may be formed through the housing between the two electrodes in the housing, and the blood glucose sensor may be formed long in a longitudinal direction, so that one end of the blood glucose sensor may be inserted into the housing to be exposed to the fine channel.

In particular, preferably, an elastic pump having a hollow space therein communicating with the fine channel may be provided on the other surface of the housing.

In this case, an air outlet for communicating the inside of the hollow space to the outside may be formed in the elastic pump, and preferably, a one-way valve may be provided on the air outlet to allow air to pass from the inside to the outside but not from the outside to the inside.

In this case, a curved surface may be preferably formed on the one surface of the housing in a shape depressed toward the elastic pump between the two electrodes, and the fine channel may be arranged at a center of the curved surface.

In addition, an air permeable membrane for passing the air but not liquid may be preferably provided between the fine channel and the hollow space inside the elastic pump.

In addition, each electrode may be preferably a flexible electrode made of a resin of a flexible material with uniformly diffused conductive particles.

A wearing band for wearing on a body may be preferably coupled to the housing.

Meanwhile, the blood glucose measurement system according to the present invention includes: a blood glucose measurement device according to any one of claims 1 to 10; a communication module provided in the blood glucose measurement device to transmit blood glucose measurement data; a server for computing and storing the blood glucose measurement data received from the communication module; and a mobile terminal for receiving the blood glucose measurement data from both of the communication module and the computing and storing module of the server and displaying the blood glucose measurement data as an image.

Here, the computing and storing module or the mobile terminal may preferably compute both a rate of change in blood glucose for a predetermined period and a time when to take a blood glucose control drug by using the received blood glucose measurement data, and may display the rate and the time on the mobile terminal.

### Advantageous Effects

In the blood glucose measurement device according to the present invention, blood glucose measurement is not performed with blood collection, but performed with glucose contained in sweat secreted due to electrical stimulation using electrodes, so as not to cause pain, infection, and scars, which are caused by a needle, and the drugs that may affect the normal sweat-inducing mechanism in the skin are not used, so as not to leave any sequelae on the skin, and even when a small amount of sweat is secreted, the sweat is efficiently collected, so as to allow all the collected sweat to be used to measure the blood glucose with high-accuracy, whereby there is an effect in that frequent blood glucose monitoring, which is important for diabetics, may be performed without sequelae.

### Description of Drawings

FIG. 1 is a view showing a blood glucose measurement scene using a conventional blood collection method.
FIG. 2 is a view showing the related art,
FIG. 3 is a front cross-sectional view showing a blood glucose measurement device according to the present invention,
FIG. 4 is a perspective plan view showing the blood glucose measurement device according to the present invention,
FIG. 5 is a view showing a structure of the human skin.
FIGS. 6a to 6c are flowcharts of an operation of the blood glucose measurement device according to the present invention.
FIG. 7a is a conceptual view showing the blood glucose measurement device and an electrode organization according to the present invention.
FIG. 7b is a sequential enlarged view of an electron micrograph showing the electrode of the present invention.
FIG. 8 is a front cross-sectional view showing an additional exemplary embodiment of FIG. 3.
FIG. 9 is a conceptual view showing a blood glucose measurement system according to the present invention.

### Best Mode

Hereinafter, the present invention will be described in detail with reference to the accompanying drawings.

As shown in FIG. 3, the present invention includes: a sweat-inducing part having electrodes; a housing 20 for fixing the sweat-inducing part; and a blood glucose sensor 30 inserted into the housing 20.

In the electrode 10 constituting the sweat-inducing part, two electrodes 10 to which opposite polarities are applied are arranged to be spaced apart from each other at a predetermined interval. Accordingly, one of two electrodes 10 corresponds to an anode, and the other electrode 10 corresponds to a cathode. The two electrodes 10 are in contact with the skin and an electric current flows between the electrodes 10 through the skin. At this time, the electric current flowing through the skin due to the electrodes 10 induces the sweating in the skin.

There are two types of sweat glands that secrete sweat: eccrine sweat glands and apocrine sweat glands. In humans, in order to secrete sweat, eccrine sweat glands are distributed throughout the skin except for areas around the armpits, navel, and genitals.

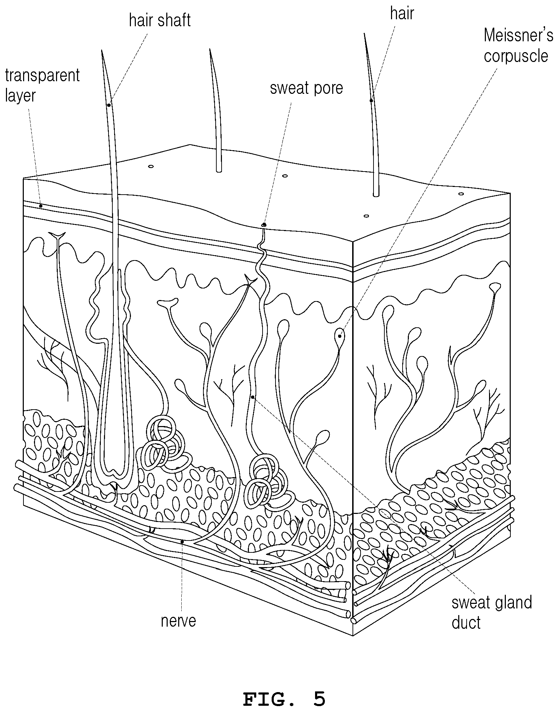

Eccrine sweat glands, which are general glands, shown in FIG. 5 act as an excretory organ, like the kidneys, that excretes waste materials. In particular, waste materials filtered from the blood of capillaries are sent along with water to the eccrine sweat glands to generate sweat, and then sweat D is discharged through the pores on the skin surface. Accordingly, glucose contained in the blood is secreted by sweat, and the blood glucose content in the sweat may be measured.

In order for sweat D to be discharged from eccrine sweat glands, stimulation to the sudomotor nerve that controls eccrine sweat glands is required. In the normal activity situation, in the process of secreting sweat D, acetylcholine is first secreted to stimulate the sudomotor nerve, and then the stimulated sudomotor nerve stimulates eccrine sweat glands. Using such a principle, in the present invention, the sudomotor nerve is stimulated by allowing an electric current of the electrodes 10 to flow, instead of applying acetylcholine, so as to cause the eccrine sweat glands to secrete sweat D.

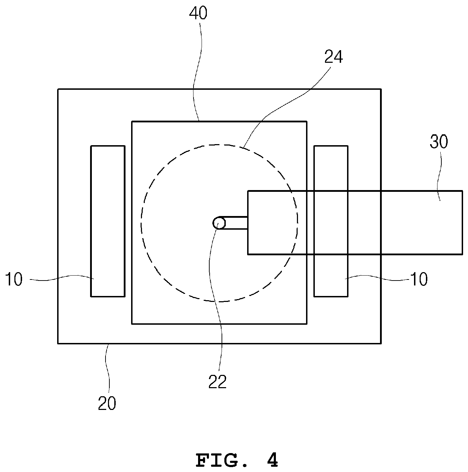

The electrodes 10 are arranged at regular intervals as shown in the plan view of FIG. 4. When an area of the electrode 10 in contact with the skin S is too small, pain may be caused to the skin S, so the electrode 10 is manufactured to have a predetermined area. The reason is that when the area of the electrode 10 is formed too small, in a condition of applying electrical stimulation, the pain caused to the skin S increases. Accordingly, after the electrode 10 is formed in more than the predetermined area, and as the electrode 10 is in close contact with the skin S over the entire area of the electrode 10, the pain caused to the skin S disappears and the electric current may flow more reliably to the skin S between the electrodes 10.

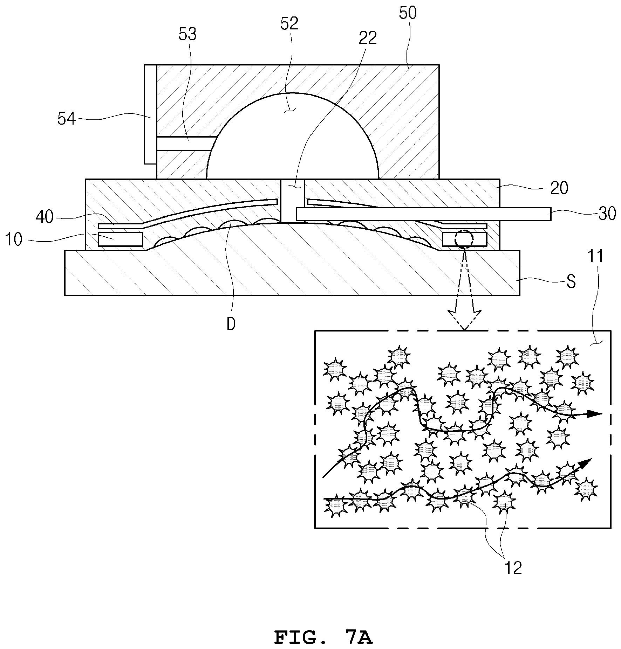

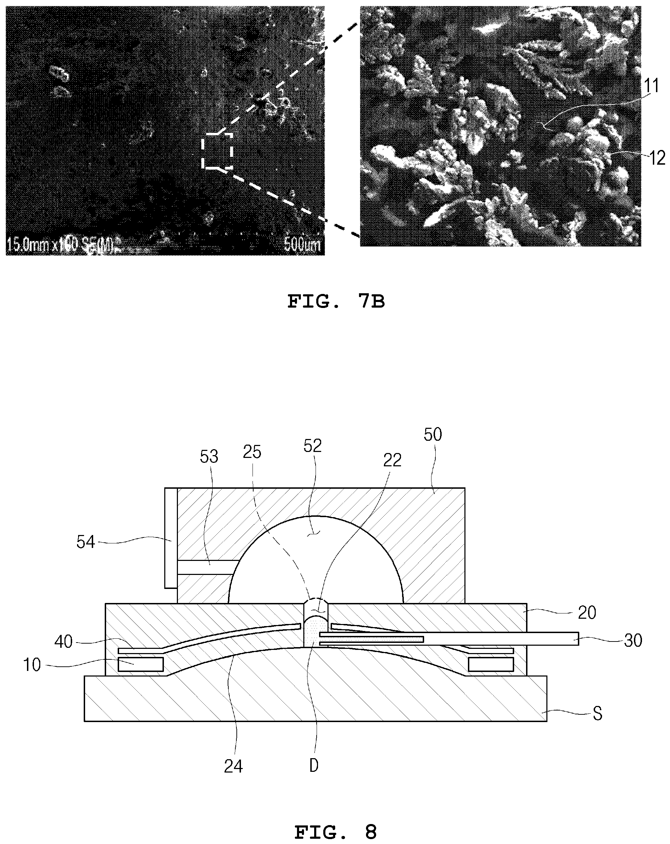

Accordingly, the electrode 10 in the present invention may be made of a flexible material so that the electrode 10 may be more reliably positioned in close contact with the skin S. As a flexible material and a material capable of ensuring an electric current to flow, as shown in FIGS. 7a and 7b, each electrode 10 in the present invention preferably uses a conductive flexible electrode made of a resin 11 of a flexible material with uniformly diffused conductive particles 12, such as metal or carbon nanofiber particles, inside a substrate of the resin 11.

FIG. 7a is a conceptual view showing a principle in which the conductive particles 12 are uniformly diffused into a resin so as to allow the electric current to continue to flow between the conductive particles 12 without being interrupted. In this case, a distance between the conductive particles 12 through which the electric current is able to flow may be determined by the density of the conductive particles 12 diffused in the resin 11. Strictly, in this case, the conductive particles 12 distributed in the resin 11 are not in close contact with each other, and are distributed at minute intervals as shown in FIG. 7b, so that the electric current flows between the conductive particles along a path formed by the conductive particles 12 having the smallest possible distance between adjacent conductive particles 12 when viewed in the electric current flow direction as shown in the conceptual view on the bottom right side of FIG. 7a.

In addition, in the present invention, a heater 40 for causing sweating may be provided together with the electrodes 10 constituting a sweat-inducing part, so that an amount of sweat D secreted by the sweating promotion function of the electrode 10 may be secured as much as the amount required for blood glucose measurement with guaranteed accuracy.

As shown in FIG. 3, the heater 40 is inserted into the interior of the housing 20, which will be described later, and is installed on a side close to the skin S.

The heater 40 may has a micro heating wire, or a carbon film, or a PTC element, or a Peltier element. In addition, the heater 40 may share the same power supply as that of the electrode 10 constituting the sweat-inducing part, and their circuits may be connected to each other.

FIG. 3 shows the housing 20 configured to be a plate shape that is widely formed to directly cover the skin S. The housing 20 fixes the sweat-inducing part, and the electrodes 10 constituting the sweat-inducing part are arranged in close contact with the skin S in FIG. 3 and fixed to contact the skin S.

In FIG. 3, a blood glucose sensor 30 is coupled to the housing 20 in a way of being inserted into an insertion hole 23 that is formed on the right side of the housing 20. At this time, as shown in FIG. 3, the housing 20 is provided with a fine channel 22 in a direction perpendicular to the center between the electrodes 10 so that sweat D secreted from a surface of the skin S under the housing 20 may contact a first end of the blood glucose sensor. The wall of the fine channel 22 is formed to communicate with the insertion hole 23 into which the blood glucose sensor 30 is inserted, whereby the first end of the blood glucose sensor 30 may contact the collected sweat D.

However, since the sweat D is secreted from the skin S due to electrical stimulation, the amount of secretion is small. In order for most of the sweat D secreted in a small amount in this way to be used to measure blood glucose, it is preferable to provide a means for suctioning the secreted sweat D to be concentrated into the fine channel 22.

In the present invention, as shown in FIG. 3, an elastic pump 50 may be provided on an upper part of the fine channel 22, so that high-accuracy blood glucose measurement may be sufficiently performed even with the sweat D secreted in a small amount.

The elastic pump 50 has a hollow space 52 formed therein, and as the hollow space 52 is contracted and then expanded, the shape of the hollow space 52 is restored to its original shape, whereby a vacuum is generated between the skin S and an area between the electrodes 10 in the housing 20, and thus the sweat D secreted from the skin S may be concentrated into the fine channel 22.

In particular, when sweat D is secreted by electrical stimulation as in the present invention, the secreted amount is as small as about 5 *µℓ*. In order to measure blood glucose with high accuracy using such a small amount of sweat D, it is necessary to supply all of the secreted sweat D to the blood glucose sensor 30.

To this end, in the elastic pump 50, the hollow space 52 formed therein communicates with a cavity between the housing 20 and the skin S through the fine channel 22, the cavity being provided between the two electrodes 10. As the elastic pump 50 is contracted and then expanded, the inside of the hollow space 52 formed in the elastic pump 50 is restored to its shape, thereby suctioning the sweat D collected between the skin S and the housing 20.

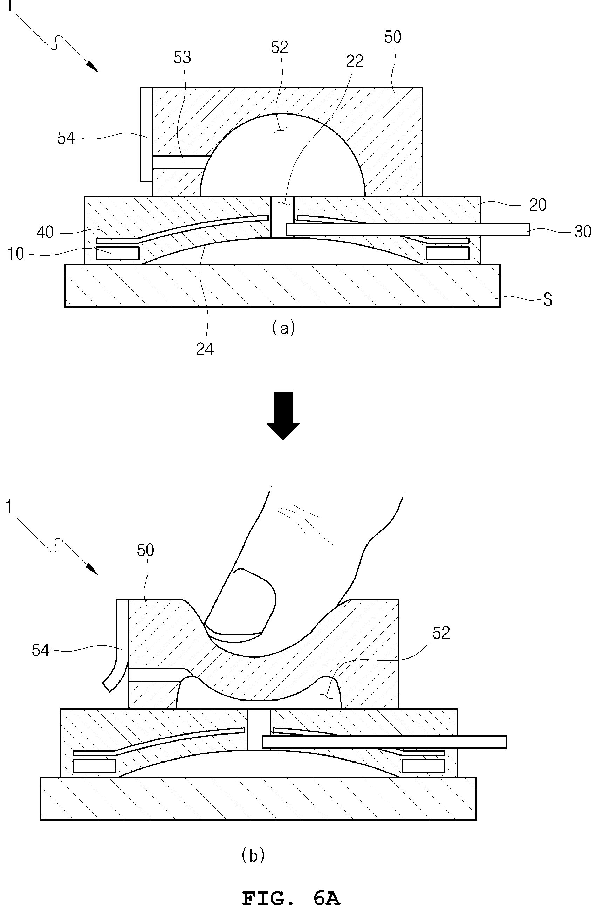

In particular, the elastic pump 50 may be provided with an air outlet 53 and a one-way valve 54 on a side surface thereof, as shown in FIG. 6a(b). In this way, as in FIG. 6a(b), when the elastic pump 50 is contracted, the air originally in the hollow space 52 inside the elastic pump 50 is not blown into the skin S direction through the fine channel 22, but escapes to the outside through the air outlet 53. In addition, when a finger is removed from the elastic pump 50, the elastic pump 50 returns to its original shape, and at this time, in the process of restoring the volume of the hollow space 52 inside the elastic pump 50 again, the external air is blocked due to the one-way valve 54 and may not enter the air outlet 53, whereby only the sweat D formed between the skin S and the housing 20 is collected while gathering in the fine channel 22.

In the front cross-sectional view shown in FIGS. 6a (a) to 6c, the principle in which sweat D is collected and blood glucose is measured according to the operation of the elastic pump 50 is sequentially expressed.

FIG. 6a(a) is the same view as FIG. 3, showing a scene in which the electrodes 10 stimulate the skin S nerve and the heater 40 starts to operate simultaneously together with the electrodes 10.

FIG. 6a (b) shows a scene in which the elastic pump 50 is contracted while the electrodes 10 and the heater 40 are operated.

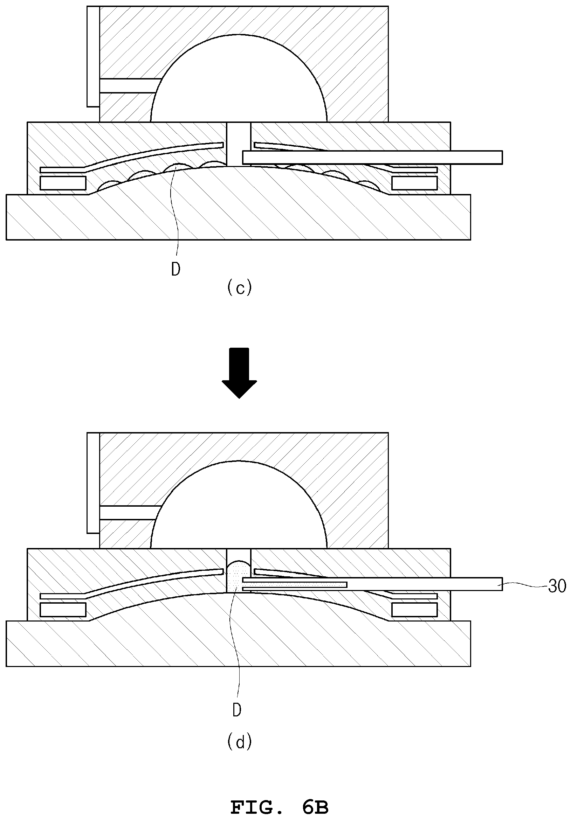

FIG. 6b(c) is a view showing a scene in which sweat D is formed on the skin S by the operation of the electrodes 10 and the heater 40, and FIG. 6b(d) is a view showing a scene in which the sweat D formed in FIG. 6b(c) is gathered into the fine channel 22 due to the operation of the elastic pump 50.

For reference, it is more appropriate that FIG. 6a(b) substantially showing the operation of the elastic pump 50 is arranged between FIG. 6b(c) and FIG. 6b (d), but in order to clearly express the operation of the elastic pump 50 and the one-way valve 54, for convenience, FIG. 6a(b) is first shown before FIG. 6b(c) and FIG. 6b (d), unlike the actual sequence.

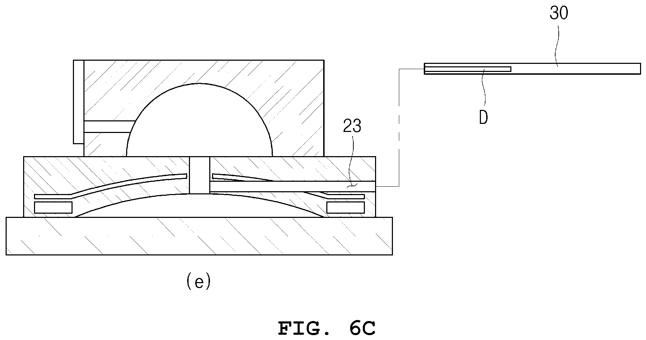

As shown in FIG. 6b (b), FIG. 6c (e) shows a scene in which the blood glucose sensor is separated from the housing 20, in order to be replaced after the blood glucose measurement is completed.

In particular, in the present invention, as shown in FIGS. 3, 6a (a) to 6c (e), 7a, and 8, among surfaces in contact with the skin S in the housing 20, a part of the surface between the electrodes 10 may be formed as a curved surface 24. As shown in FIG. 3, the curved surface 24 has a curved shape in the form of an arch facing upward.

The reason why the curved surface 24 is formed in this way is that since the area of a surface in the housing 20 in contact with the skin S is larger when the surface is curved than when the surface is flat, when the surface of the housing 20 in contact with the skin S, that is, the surface of the housing 20 between the electrodes 10, is formed in a curved shape, the area of the skin S in contact with the housing 20 is increased, so that a greater amount of sweat D is collected into the fine channel 22, whereby the greater amount of sweat D may be secured for more accurate blood glucose measurement.

Meanwhile, as shown in FIG. 8, in the present invention, an air permeable membrane 25 through which gas may pass but liquid does not pass may be provided at a position between the hollow space 52 and the fine channel 22 of the elastic pump 50, so as to prevent a phenomenon where sweat D is suctioned into the hollow space 52 inside the elastic pump 50 due to a large suction force of the elastic pump 50 when the sweat D is concentrated in the fine channel 22 by the operation of the elastic pump 50.

In other words, the reason is that since the entire amount of sweat D should be used for the purpose of measuring blood glucose, the fact that sweat D is absorbed into the hollow space 52 inside the elastic pump 50 may result in problems in that unnecessary loss of sweat D occurs and also the inside of the elastic pump 50 needs to be cleaned more often.

Meanwhile, as shown in FIGS. 3, 6c (e), and 8, the blood glucose sensor has a long groove formed from one end thereof to a predetermined length so that sweat D may be quickly absorbed, whereby it may be possible to promote the absorption of the sweat D and check an absorption amount of the sweat D. The absorption amount of sweat D is an important data to check a generated amount of sweat D, and in the case of diabetic patients, the generated amount of sweat may be significantly reduced in comparison with normal people due to peripheral nerve paralysis, so that the absorption amount of sweat D may be used as data for self-diagnosis of diabetes.

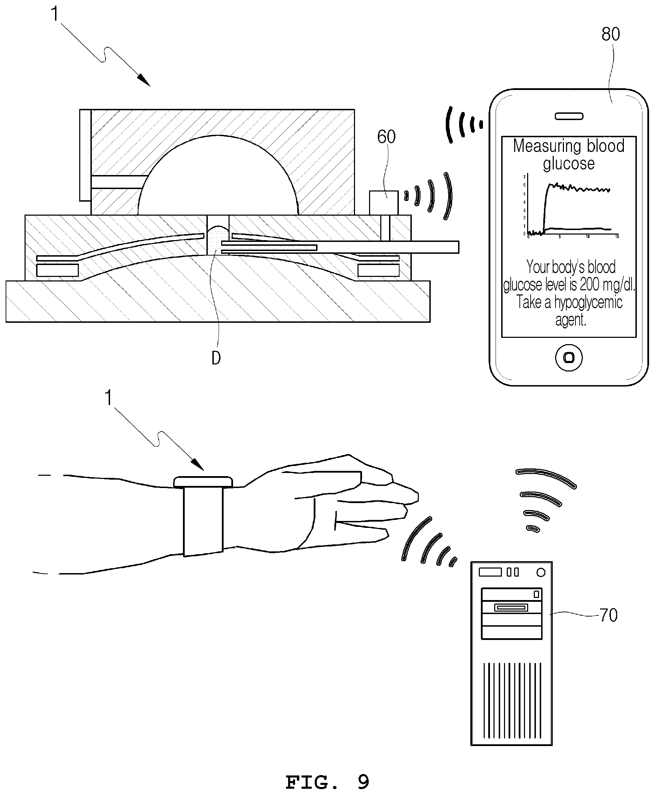

In addition, as shown in FIG. 9, in the blood glucose measurement device according to the present invention, a wearing band is connected to the housing 20 so as to be worn on the wrist, whereby blood glucose may be measured while wearing the wearing band on the wrist whenever necessary.

In FIG. 9, it is not shown in detail whether the blood glucose measurement device 1 according to the present invention is worn on the wrist, but a device positioned on an upper part of the wrist is the blood glucose measurement device 1 according to the present invention and a wearing band is wrapped around the wrist.

Hereinafter, a blood glucose measurement system according to the present invention will be described.

As shown in FIG. 9, the blood glucose measurement system according to the present invention is configured to include: a blood glucose measurement device 1 configured as described above; a communication module 60 provided in the blood glucose measurement device 1 to transmit blood glucose measurement data; a server 70 for computing and storing the blood glucose measurement data received from the communication module 60; and a mobile terminal 80 for receiving the blood glucose measurement data from the communication module 60 and server 70, and displaying the data as an image.

The mobile terminal 80 in this case may be typically a smart phone or a mobile smart device combined with the blood glucose measurement device 1 itself.

In addition, the mobile terminal 80 by itself may compute a blood glucose measurement value detected by the blood glucose measurement device and display the value in a graph so that a trend of blood glucose increase and decrease by time may be displayed at a glance as shown in FIG. 9. Alternately, the server 70 in a remote area may compute the blood glucose measurement value, so as to allow information, indicating a rate of change in blood glucose and a time when to take a blood glucose control drug according to the rate of change in blood glucose, to be displayed on the mobile terminal 80.

In this case, the server 70 in the remote area may show the rate of change in blood glucose over a long period of time. In other words, it is possible to show blood glucose measurement values measured at a specific time every day, on a weekly, monthly, or quarterly basis. Accordingly, it is possible to make a recommendation of whether to administer blood glucose control drugs and to change the amount of drug administration, and the recommendation regarding the drug administration may be displayed on the mobile terminal 80.

Such a server 70 in the remote area is also connected to a hospital through a network so that an attending physician may inquire the information as necessary, whereby the attending physician may directly contact a patient and deliver appropriate measures according to a sudden change in blood glucose even without the patient visiting the hospital. In addition, the mobile terminal 80 may be provided with an input function installed therein, so that data input to the mobile terminal 80 may be transmitted to the server 70.

The present invention described above is not limited by the above-described exemplary embodiments and the accompanying drawings, and obviously, those skilled in the art will appreciate that various substitutions, modifications, and changes are possible within the scope of the technical spirit of the present invention.

### <Description of the Reference Numerals in the Drawings>

| | |
|---|---|
| 1: blood glucose measurement device according to the present invention | |
| D: sweat | S: skin |
| 10: electrode | 11: resin |
| 12: conductive particles | 20: housing |
| 22: fine channel | 23: insertion hole |
| 24: curved surface | 25: air permeable mem-brane |
| 30: blood glucose sensor | 40: heater |
| 50: elastic pump | 52: hollow space |
| 53: air outlet | 54: one-way valve |
| 60: communication module | 70: server |
| 80: mobile terminal | |

## Claims

1. A blood glucose measurement device comprising:
a sweat-inducing part having two electrodes arranged to be spaced apart from each other and to which opposite polarities are respectively applied;
a housing for fixing the sweat-inducing part allowing the two electrodes to be exposed on one surface of the housing; and
a blood glucose sensor inserted into the housing and in contact with body fluids induced by the sweat-inducing part.

2. The blood glucose measurement device of claim 1, wherein a heater is installed inside the housing close to the one surface of the housing, and
the heater has a carbon film, or a micro hot wire, or a Peltier element, or a PTC element.

3. The blood glucose measurement device of claim 1, wherein the blood glucose sensor is detachably inserted into the housing from an outer side of the housing.

4. The blood glucose measurement device of claim 1, wherein a fine channel is formed through the housing between the two electrodes in the housing,
an insertion hole is formed long penetrating a sidewall of the fine channel and one side of the housing, and
the blood glucose sensor is formed long in a longitudinal direction, so that the blood glucose sensor is inserted into the insertion hole, whereby one end of the blood glucose sensor is exposed to the fine channel.

5. The blood glucose measurement device of claim 4, wherein an elastic pump having a hollow space therein communicating with the fine channel is provided on the other surface of the housing.

6. The blood glucose measurement device of claim 5, wherein an air outlet for communicating the inside of the hollow space to the outside is formed in the elastic pump, and
a one-way valve is provided on the air outlet to allow air to pass from the inside to the outside but not from the outside to the inside.

7. The blood glucose measurement device of claim 6, wherein a curved surface is formed on the one surface of the housing in a shape depressed toward the elastic pump between the two electrodes, and
the fine channel is arranged at a center of the curved surface.

8. The blood glucose measurement device of claim 6, wherein an air permeable membrane for passing the air but not liquid is provided between the fine channel and the hollow space inside the elastic pump.

9. The blood glucose measurement device of claim 1, wherein each electrode is a flexible electrode made of a resin of a flexible material with uniformly diffused conductive particles.

10. The blood glucose measurement device of claim 1, wherein a wearing band for wearing on a body is coupled to the housing.

11. The blood glucose measurement device of claim 4, wherein the blood glucose sensor has a long groove formed therein by a predetermined length from the one end thereof so as to quickly absorb sweat, thereby being able to promote sweat absorption and check an absorption amount of the sweat.

12. A blood glucose measurement system using a blood glucose measurement device according to any one of claims 1 to 11, the blood glucose measurement system comprising:
the blood glucose measurement device;
a communication module provided in the blood glucose measurement device to transmit blood glucose measurement data;
a server for computing and storing the blood glucose measurement data received from the communication module; and
a mobile terminal for receiving the blood glucose measurement data from the communication module and the server and displaying the blood glucose measurement data as an image.

13. The blood glucose measurement system of claim 12, wherein the server or mobile terminal computes both a rate of change in blood glucose for a predetermined period and a time when to take a blood glucose control drug by using the received blood glucose measurement data and displays the rate and the time on
the mobile terminal.
